# EUROPEAN PATENT SPECIFICATION

(11) **EP 3 908 359 B1**
(45) Date of publication and mention of the grant of the patent: **18.10.2023**
(21) Application number: 20739205.1
(22) Date of filing: 07.01.2020
(51) Int. Cl.: A61N 1/36

(54) **SYSTEM FOR DELIVERING ELECTRICAL STIMULATION**
SYSTEME ZUR VERABREICHUNG EINER ELEKTRISCHEN STIMULATION
SYSTÈME DE DÉLIVRANCE D'UNE STIMULATION ÉLECTRIQUE

(30) Priority: 07.01.2019 US 201962789236 P
(43) Date of publication of application: 17.11.2021
(73) Proprietor: Halo Neuro, Inc., San Francisco, CA 94103 (US)
(72) Inventor: WINGEIER, Brett, San Francisco, CA 94103 (US)
(74) Representative: Murgitroyd & Company
(86) International application number: PCT/US2020/012588
(87) International publication number: WO 2020/146392

(56) References cited:
- WO-A1-2013/022840
- US-A1- 2007 027 498
- US-A1- 2014 148 872
- US-A1- 2015 005 840
- US-A1- 2017 224 991
- US-A1- 2017 224 991

## Description

### TECHNICAL FIELD

This invention relates generally to the neuromodulation field, and more specifically to a new and useful system and method for delivering electrical stimulation in the neuromodulation field.

### BACKGROUND

Current systems and methods in the neuromodulation field are used to transmit electrical signals to a subject, which has been shown to improve a user's performance of various tasks, as well as in various fields and applications, such as: consumer use, clinical trials, and research. The types of tasks and fields in which neuromodulation can have a valuable effect are constantly expanding, and current systems and methods for providing electrical stimulation are inadequate in providing electrical stimulation for various different applications which is still continuous, robust, and able to efficiently and accurately receive adjustments.

Thus, there is a need in the neuromodulation field to create an improved and useful system and method for reliably and continuously applying various types of electrical stimulation to a user. US 2017/224991 discloses related art.

### BRIEF DESCRIPTION OF THE FIGURES

FIGURE 1 is a schematic of a system for providing electrical stimulation.
FIGURE 2 is a schematic of a method for providing electrical stimulation.
FIGURE 3 is a schematic of a variation of the method for providing electrical stimulation.
FIGURE 4 is a schematic of a variation of the system for providing electrical stimulation.
FIGURES 5A and 5B depict a variation of a client application.
FIGURE 6 depicts a first variation of an electrical stimulation device.
FIGURE 7 depicts a second variation of an electrical stimulation device.
FIGURES 8A-8F depict a variation of a method for providing sham stimulation to a user.

### DESCRIPTION OF THE PREFERRED EMBODIMENTS

The invention is defined in the appended claims. Aspects, embodiments and examples disclosed herein which do not fall within the scope of the appended claims do not form part of the invention and are merely provided for illustrative purposes. Furthermore, the methods presented in the present description are not claimed as such but are useful for understanding of the claimed subject matter, in particular since the methods can be performed by the claimed subject-matter. Z

### 1. Overview

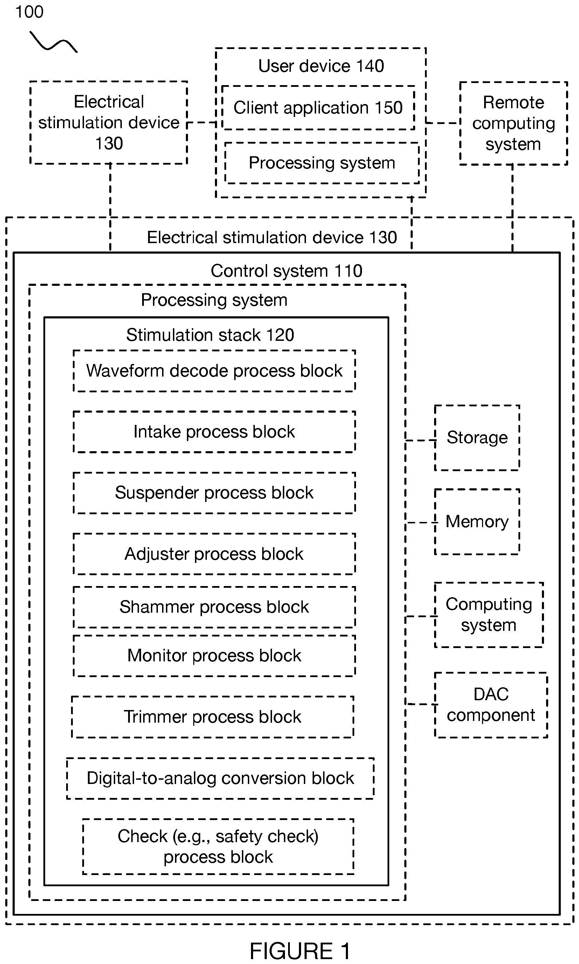

As shown in FIGURE 1, a system 100 for delivering electrical stimulation includes a control system 110 and a stimulation stack 120. Additionally or alternatively, the system can include and/or be configured to interface with any or all of: an electrical stimulation device 130, a user device 140, a client application 150, processing system, storage, memory, a computing system, a remote computing system, a digital-to-analog converter (DAC) component, and/or any other suitable components. Further additionally or alternatively, the system can include or all of the components as described in any or all of: U.S. Patent Application Number 15/426,212, filed 07-FEB-2017, U.S. Patent Application Number 15/916,170, filed 08-MAR-2018, U.S. Patent Application Number 14/878,647, filed 08-OCT-2015, and U.S. Patent Application Number 15/335,240, filed 26-OCT-2016.

The system 100 can be performed in accordance with the method 200 described below and/or with any other suitable method.

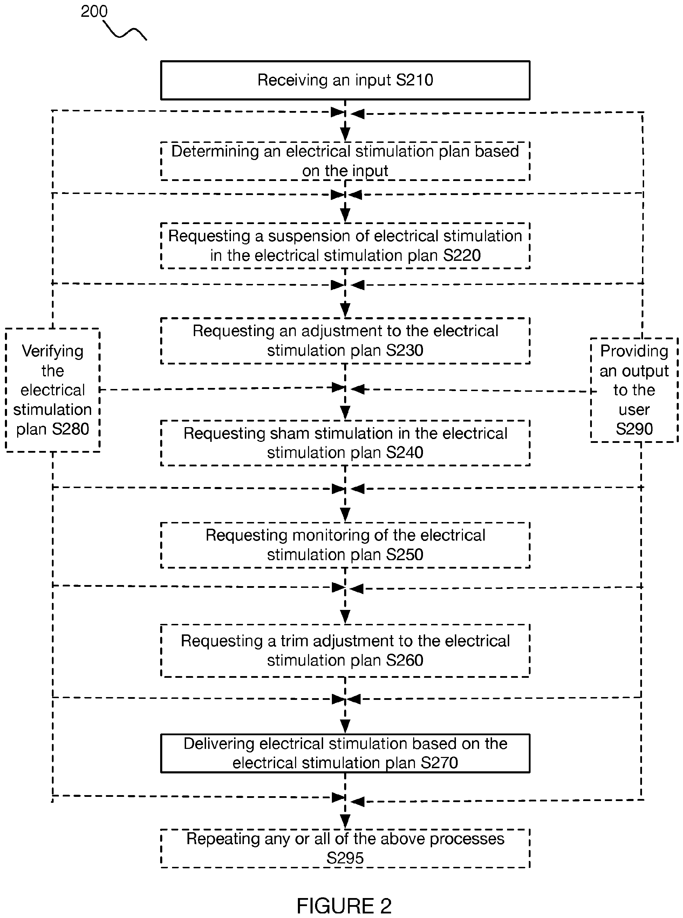

As shown in FIGURE 2, a method 200 for delivering electrical stimulation includes receiving an input S210 and delivering electrical stimulation based on an electrical stimulation plan S270. Additionally or alternatively, the method 200 can include any or all of: determining an electrical stimulation plan based on the input; requesting a suspension of the electrical stimulation plan S220, requesting an adjustment to the electrical stimulation plan S230; requesting sham stimulation in the electrical stimulation plan S240; requesting monitoring of the electrical stimulation plan S250; requesting a trim adjustment to the electrical stimulation plan S260; checking the electrical stimulation plan S280; providing an output to the user S290; repeating any or all of the above processes S295; and/or any other suitable processes. Further additionally or alternatively, the method 200 can include any or all of the processes described in any or all of: the Patent Applications described above; U.S. Patent Application Number 14/470,683, filed 27-AUG-2014;
U.S. Patent Application Number 14/470,747, filed 27-AUG-2014; U.S. Patent Application Number 15/426,212, filed 07-FEB-2017;
and/or any other suitable processes performed in any suitable order.

The method 200 can be performed with a system as described above and/or any other suitable system.

### 2. Benefits

The system and method for delivering electrical stimulation can confer several benefits over current systems and methods.

In a first variation, the system and/or method confers the benefit of continuously monitoring and accurately adjusting (e.g., in real time, in near real time, etc.) electrical stimulation being applied to the user. In a specific example, for instance, the system and method continuously monitor for adjustments (e.g., from adjustment requests) to be applied to the electrical stimulation (e.g., as triggered by user input, as required to maintain a set of safety standards, etc.) and implement the adjustments through an expected yet safe process.

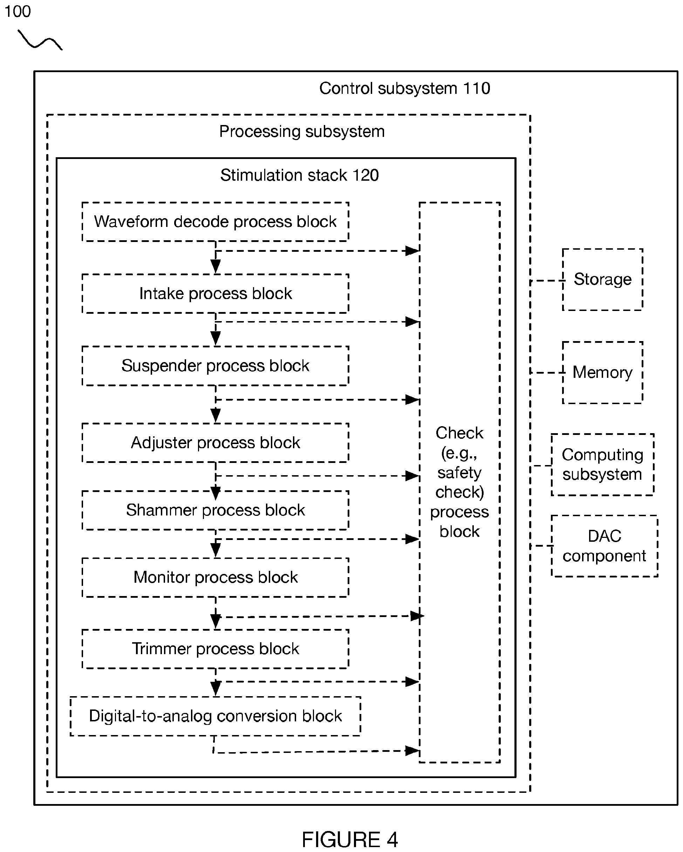

In a second variation, the system and/or method confers the benefit of robustly providing complex electrical stimulation to a user. In a specific example, for instance, the system and method apply transcranial alternating current stimulation through a specific ordered sequence of stimulation stack process blocks (e.g., as shown in FIGURE 4), wherein the order is configured for any or all of preventing a system crash while dealing with complex waveforms (e.g., transcranial alternating current stimulation, transcranial random noise stimulation, etc.), preventing a user from discovering that he or she is receiving sham stimulation, and appropriately applying adjustments to a stimulation waveform being applied to the user.

In a third variation, the system and/or method confers the benefit of providing sham stimulation to a user without him or her knowing that sham stimulation is being applied, especially in edge case situations which might reveal that sham is being applied. In a specific example, in the event that the stimulation device loses contact with the user, the system can still indicate (e.g., through a notification at a client application) to the user that stimulation has stopped.

In a fourth variation, the system and/or method confers the benefit of compensating for various hardware limitations which may be integrated into the system hardware. In a specific example, for instance, the system and method enable a cost-effective digital-to-analog (DAC) converter component to be used by adjusting the electrical stimulation (e.g., with a trimming process block) prior to being applied to the user to counteract the particular limitation.

Additionally or alternatively, the system and method can confer any other benefits.

### 3. System 100

The system 100 functions to apply electrical stimulation to a user. Additionally or alternatively, the system 100 can function to operate in a sham operation mode (e.g., for use in a clinical trial, for use in a research study, etc.), equivalently referred to herein as a sham stimulation mode; continuously monitor electrical stimulation being applied to the user; and/or perform any other suitable function(s).

The system 100 is preferably configured to apply transcranial electrical stimulation, further preferably all of: transcranial direct current stimulation (tDCS), transcranial alternating current stimulation (tACS), transcranial magnetic stimulation (TMS), transcranial random noise stimulation (tRNS) (e.g., band-limited random noise stimulation), transcranial pulsatile stimulation (tPS), transcranial variable frequency stimulation (tVFS), band-limited stimulation transformed to increase RMS power while minimizing transients and clipping, and/or any other form of transcranial stimulation. Additionally or alternatively, the system 100 can be configured to apply a subset of these (e.g., only direct current stimulation, only alternating current stimulation, etc.), additional forms of stimulation, and/or any other suitable form of electrical stimulation.

The electrical stimulation is preferably applied through a set of one or more electrical stimulation sessions, which are prescribed by an electrical stimulation plan, wherein each electrical stimulation session defines a set of one or more stimulation waveforms. The waveforms can be defined by any or all of the following parameters: a current amplitude (e.g., maximum amplitude, minimum amplitude, average amplitude, etc.), a frequency, a waveform function or type (e.g., a square wave, a sine waveform, a cosine waveform, a superposed waveform, etc.), a voltage, a power, an energy, temporal parameters (e.g., time of delivery, duration of stimulation, etc.), and/or any other suitable parameters. Additionally or alternatively, electrical stimulation can be applied through any suitable stimulation defined by any suitable set of parameters.

The system 100 includes a control system 110, which functions to control provision and adjustment of an electrical stimulation session (e.g., set of one or more waveforms associated with the electrical stimulation session) in accordance with a set of process blocks (e.g., as described below). The control system 110 can be part of any or all of: the electrical stimulation device (e.g., onboard the stimulation device), a user device separate and distinct from the electrical stimulation device (e.g., as described below), a remote control subsystem, and/or any other suitable component(s) or combination of components (e.g., distributed among a processor of a user device and a processor onboard the electrical stimulation device). In a first variation, the control system 110 is arranged onboard the electrical stimulation device. In a second variation, the control system 110 is distributed among the electrical stimulation device and a user device (e.g., via a client application), wherein a portion of the processing is performed onboard the electrical stimulation device and a portion of the processing is performed at the user device. Further additionally or alternatively, any of all of the control system can be arranged at a remote computing system.

The control system 110 includes and/or implements a stimulation stack 120 comprising a set of process blocks (e.g., embedded process blocks, non-embedded stimulation stack, etc.), which function to define how electrical stimulation is being applied, monitored, and adjusted. The process blocks of the stimulation stack 120 are preferably arranged in a specified order which enables electrical stimulation of various types (e.g., tDCS, tRNS, etc.) to be robustly applied to the user (e.g., without causing a system crash). Additionally or alternatively, the process blocks can function to properly adjust electrical stimulation as prompted by a user and/or determined by the system 100, prevent a user from discovering that the system 100 is operating in a sham stimulation mode, and/or perform any other suitable function(s). Further additionally or alternatively, the process blocks can be arranged in any particular order configured to achieve any suitable function.

Each of the set of process blocks of the stimulation stack is preferably configured to operate based on information (e.g., working data, state information, an output, etc.) associated with one or more preceding process blocks. Additionally or alternatively, any or all the process blocks can be configured to operate based on other information, such as user input (e.g., received at a client application, received at the electrical stimulation device, prior to and/or during an adjuster system block as described below, etc.), stored information (e.g., stored at a remote lookup table, stored onboard the electrical stimulation device, stored at the user device, etc.), and/or any other suitable information. Further additionally or alternatively, the process blocks can operate based on other suitable information, in absence of information from one or more preceding blocks, in absence of user input (e.g., all blocks except the suspender and adjuster blocks, etc.), and/or otherwise operate.

The stimulation stack preferably operates according to a serial program, wherein incoming data and information are processed sequentially, one process block at a time. In some variations, process blocks deemed to be irrelevant to the electrical stimulation session are skipped. In a specific variation, for instance, only electrical stimulation sessions configured to implement a sham stimulation operation mode are processed with a shammer system block. Additionally or alternatively, the system blocks can be operated in any other suitable way according to any suitable stimulation program (e.g., according to a stateless stimulation system).

In one variation, the set of system blocks includes the following ordered system blocks: an intake block configured to implement the request for electrical stimulation (e.g., as described below); a suspender block configured to implement a request for a suspension to the electrical stimulation plan (e.g., as described below); an adjuster block configured to implement a request for adjustment to the electrical stimulation plan (e.g., as described below); a shammer block configured to implement a request for sham stimulation; a monitoring block configured to implement a request for monitoring of the electrical stimulation plan (e.g., as described below); a trimmer process block configured to prepare an electrical stimulation plan to be implemented at a particular electrical stimulation device (e.g., as described below); a digital-to-analog conversion block configured to apply electrical stimulation (e.g., one or more waveforms of the electricals stimulation session, sham stimulation, etc.) at the electrical stimulation device (e.g., as described below); a check block configured to compare planned electrical stimulation with actual electrical stimulation delivered by the electrical stimulation device; and/or any other suitable process blocks.

The control subsystem preferably includes and/or is configured to interface with a digital-to-analog converter (DAC) component, such as the DAC management component described in U.S. Patent Application Number 15/426,212, filed 07-FEB-2017, which is incorporated in its entirety herein by this reference. Additionally or alternatively, the control system can be configured to interface with another DAC component or in absence of a DAC component.

The control system no can optionally include any or all of: a processing system (e.g., including the stimulation stack), a computing system, memory (e.g., onboard the electrical stimulation device, onboard a user device, at a remote computing subsystem, etc.), storage, and/or any other suitable component(s).

The system 100 can be operable in any number of operation modes, such as any or all of: an active electrical stimulation mode, a sham stimulation mode, an on mode, an off mode, a suspended (e.g., paused) stimulation mode, and/or any other suitable mode(s).

In some variations, different versions of the system 100 can vary in the option to provide a sham stimulation mode and/or the what is required to implement a sham stimulation mode. This can be implemented through any or all of: hardware of the system, firmware of the system, software of the system, and/or through any other components of the system or processes of the method. In a specific example, for instance, consumer devices (e.g., as opposed to academic / clinical / research devices) are either not operable in a sham stimulation mode and/or require a sham stimulation mode option to be added (e.g., by downloading a sham stimulation software package). This can prevent users from inadvertently applying sham stimulation, resulting in disappointment with the results. Additionally or alternatively, however, the systems can be consistent among use cases and/or otherwise configured.

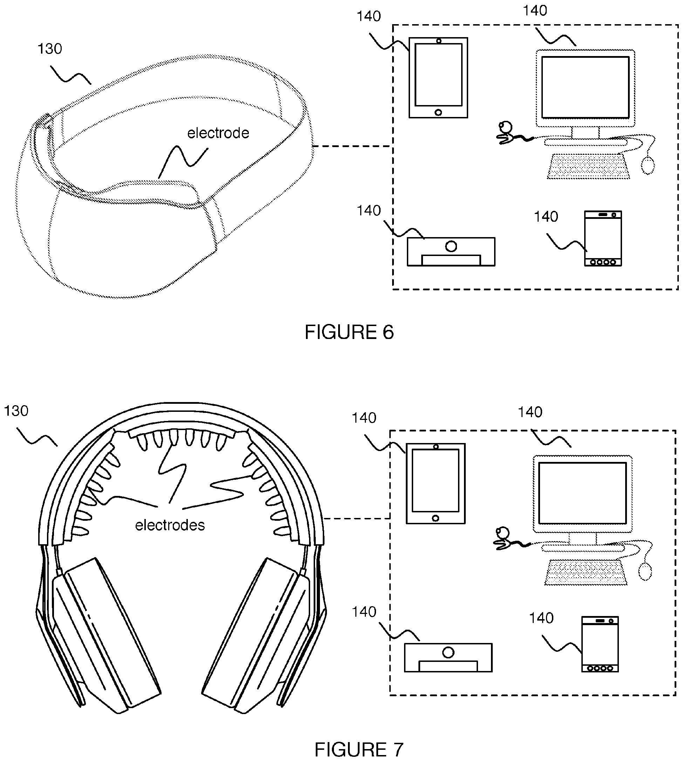

The system 100 preferably includes and/or is configured to interface with (e.g., be coupled to, be integrated within, etc.) an electrical stimulation device 130 (e.g., as shown in FIGURE 6, as shown in FIGURE 7, etc.), which can include any or all of the variations, embodiments, and examples described in any or all of: U.S. Patent Application Number 15/916,170, filed 08-MAR-2018, U.S. Patent Application Number 14/878,647, filed 08-OCT-2015, and U.S. Patent Application Number 15/335,240, filed 26-OCT-2016.

The system 100 can include and/or be configured to interface with a user device 140, wherein the user device 140 functions to execute one or more client applications 150. Additionally or alternatively, the user device 140 can function to perform part or all of the processing of the system, receive and/or store data from the system (e.g., from the usage module), provide an interface with a remote computing subsystem and/or remote storage, and/or perform any other suitable function.

The user device 140 is preferably separate and distinct from the electrode assembly and head apparatus, but can additionally or alternatively be integrated with one of these components or otherwise arranged.

Examples of the user device 140 include a tablet, smartphone, mobile phone, laptop, watch, wearable device (e.g., glasses), or any other suitable user device. The user device can include power storage (e.g., a battery), processing systems (e.g., CPU, GPU, memory, etc.), user outputs (e.g., display, speaker, vibration mechanism, etc.), user inputs (e.g., a keyboard, touchscreen, microphone, etc.), a location system (e.g., a GPS system), sensors (e.g., optical sensors, such as light sensors and cameras, orientation sensors, such as accelerometers, gyroscopes, and altimeters, audio sensors, such as microphones, etc.), data communication system (e.g., a WiFi transceiver(s), Bluetooth transceiver(s), cellular transceiver(s), etc.), or any other suitable component.

The system 100 can additionally include and/or be configured to interface with one or more client applications 150 (e.g., which execute on a user device 140). A client application 150 functions to provide an interface with the user (e.g., to receive user inputs, to provide outputs to a user, etc.). Additionally or alternatively, the client application 150 can function to process any or all of the data (e.g., number of counts, data received at tag, etc.) received at the system 100, store data (e.g., predetermined stimulation sessions to be applied through the electrode assembly, usage data, number of counts, calculation based on number of counts, cyclic redundancy check calculation, etc.), retrieve data (e.g., from a lookup table at a remote computing system), provide information and/or stimulation options to a user (e.g., type of stimulation, task options, stimulation session options, stimulation session parameters, etc.), and/or perform any other suitable function.

The client application 150 can optionally check for an electrode assembly being present before initiating and/or continuing with an already initiated electrical stimulation session. Additionally or alternatively, an orientation of the electrode assembly can be checked, a number of electrodes can be checked, a position and/or location of the electrodes can be checked, this can be not checked, any information can be checked at a processing subsystem onboard the head apparatus, and/or any other suitable information can be checked at any suitable component(s) of the system.

The client application 150 can optionally visually guide the user (e.g., by indicating on a graphic display of the user device) in properly placing a set of electrodes (e.g., upon determining that the electrodes have been placed incorrectly, any time the user is coupling electrodes to a head apparatus of the electrical stimulation device, etc.), guide the user through audio (e.g., telling the user that the electrodes are not properly placed, telling the user how to adjust placement for proper use, etc.), and/or otherwise assist the user in properly coupling a set of electrodes with a head apparatus.

In one variation, the system includes a control subsystem configured to continuously implement a neurostimulation stack according to a serial program during active electrical stimulation modes and sham stimulation modes, wherein the control subsystem is configured to control electrical stimulation applied through an electrical stimulation device, wherein the electrical stimulation device is in communication with a user device executing a client application. In a first specific example, the system is configured to interface with the flexible electrical stimulation device configured to conform to and apply electrical stimulation to a forehead region of the user, as described in U.S. Patent Application Number 15/916,170, filed 08-MAR-2018. In a second specific example, the system is configured to interface with the headset electrical stimulation device configured to apply electrical stimulation to a motor cortex region of the user, as described in one or both of U.S. Patent Application Number 14/878,647, filed 08-OCT-2015, and U.S. Patent Application Number 15/335,240, filed 26-OCT-2016.

In one variation of the system 100, the system includes a control system, which includes a stimulation stack of embedded process blocks. The control system is at least partially arranged onboard an electrical stimulation device, which is configured to apply electrical stimulation in accordance with an electrical stimulation plan to a user through a set of electrodes. The stimulation stack preferably includes the following ordered process blocks: an intake block configured to implement the request for electrical stimulation; a suspender block configured to implement a request for a suspension to the electrical stimulation plan; an adjuster block configured to implement a request for adjustment to the electrical stimulation plan; a shammer block configured to implement a request for sham stimulation; a monitoring block configured to implement a request for monitoring of the electrical stimulation plan; a trimmer process block configured to prepare an electrical stimulation plan to be implemented at a particular electrical stimulation device; a digital-to-analog conversion block configured to apply electrical stimulation (e.g., one or more waveforms of the electricals stimulation session, sham stimulation, etc.) at the electrical stimulation device; and a check block configured to compare planned electrical stimulation with actual electrical stimulation delivered by the electrical stimulation device. Additionally or alternatively, the stimulation stack can include any other process blocks performed in any suitable order. The stimulation stack is preferably run through at a predetermined frequency (e.g., once every 0.4 milliseconds, more than once every 0.4 milliseconds, once every millisecond, more than once every millisecond, once every second, once every 10 seconds, once every minute, between once every second and once every minute, less than once every minute, more than once per second, etc.) while stimulation (or sham stimulation) is being applied to the user, but can additionally or alternatively be otherwise performed. The system is preferably configured to interface with a user device in communication with the electrical stimulation device, wherein the user device executes a client application, which can receive inputs from the user (e.g., electrical stimulation selection, parameter adjustment, etc.) and display outputs to the user.

### 4. Method 200

As shown in FIGURE 2, the method 200 includes receiving an input S210 and delivering electrical stimulation based on an electrical stimulation plan S270. Additionally or alternatively, the method 200 can include any or all of: determining an electrical stimulation plan based on the input; requesting a suspension of the electrical stimulation plan S220, requesting an adjustment to the electrical stimulation plan S230; requesting sham stimulation in the electrical stimulation plan S240; requesting monitoring of the electrical stimulation plan S250; requesting a trim adjustment to the electrical stimulation plan S260; checking the electrical stimulation plan S280; providing an output to the user S290; repeating any or all of the above processes S295; and/or any other suitable processes.

The method 200 functions to continuously and robustly apply electrical stimulation to a user. Additionally or alternatively, the method 200 can function to determine the electrical stimulation to be applied to the user, monitor the electrical stimulation being applied to the user, adjust the electrical stimulation that is applied to the user, apply sham stimulation to a user, prevent a user from discovering that the system is operation in a sham stimulation mode, and/or perform any other suitable function(s).

The method 200 is preferably performed with a system 100 as described above but can additionally or alternatively be performed with any other suitable system. In some variations, for instance, the method is at least partially performed with a stimulation stack 120 as described above.

The method 200 is preferably performed continuously (e.g., at a predetermined frequency, in response to a trigger, etc.) while an electrical stimulation session is being applied to the user, wherein the electrical stimulation session can include one or both of active electrical stimulation and sham stimulation. Active electrical stimulation herein refers to electrical stimulation with a substantially non-zero current amplitude; additionally or alternatively, active electrical stimulation can include any or all of continuous electrical stimulation, electrical stimulation configured to induce a positive neurological effect in a user, and/or any other suitable electrical stimulation. Sham stimulation herein refers to electrical stimulation having substantially zero current amplitude. The sham stimulation can include a non-zero current amplitude, such as at the beginning of the sham stimulation and at the end of the sham stimulation, as most users do not notice that substantially zero current is being applied during the portion in-between. Additionally or alternatively, the active electrical stimulation and the sham stimulation can include any other suitable stimulation type(s) applied for any suitable duration.

### 4.1 Method: Receiving an input S210

The method 200 can optionally include receiving an input S210, which functions to initiate an electrical stimulation session. Additionally or alternatively, S210 can function to receive a user selection of an electrical stimulation session, adjust an electrical stimulation session (e.g., adjust a stimulation parameter, increase a current amplitude, decrease a current amplitude, etc.), suspend an electrical stimulation session (e.g., in response to a user's request for a pause to the electrical stimulation, to prevent an unsafe amount of stimulation from being applied, etc.), and/or perform any other suitable function(s).

The input can be received from the user, such as at any or all of: an input (e.g., button) of the electrical stimulation device, a user interface (e.g., touch screen) associated with a user device, at a client application executing on the user device, at a sensor subsystem (e.g., microphone) associated with the system 100, and/or at any other suitable interface. The input can additionally or alternatively be generated automatically (e.g., at the system 100, at the control subsystem 110, at a remote computing system, etc.), such as in response to any or all of: a user schedule (e.g., as stored at the user device, as received at a client application, etc.), a temporal parameter (e.g., time of day, day of week, etc.), information received at a sensor subsystem (e.g., detection of system placement on user, based on data from a thermal sensor, based on data from a contact sensor, etc.), and/or otherwise automatically generated and/or received. Further additionally or alternatively, an input can be received from a software object or system block (e.g., embedded system block, previous system block in the set of system blocks 120, etc.). In some variations, for instance, one or more software objects and/or system blocks read a waveform description of the electrical stimulation session and implement instructions for output included in the waveform description.

The input of S210 can be received at any or all of: a user device (e.g., a client application executing on a user device, a user interface of the user device, etc.), at the electrical stimulation device (e.g., at one or more buttons onboard the head apparatus), at a remote computing system, and/or at any other suitable component(s) of the system.

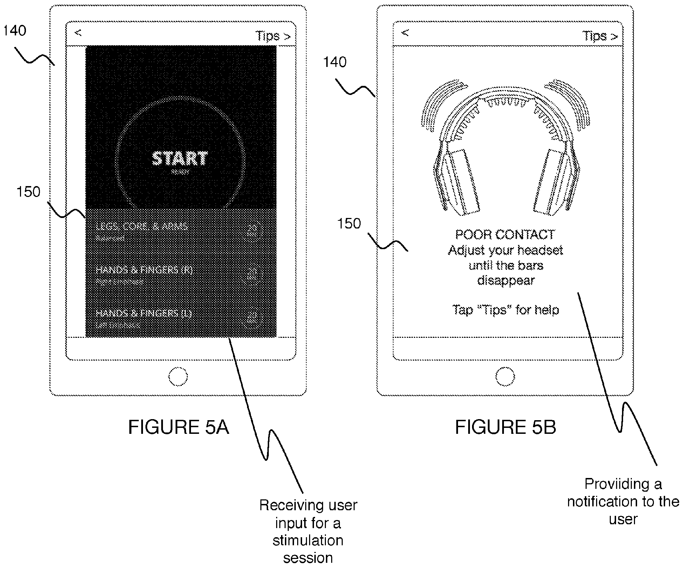

The input preferably includes a request for action from the user, which specifies a stimulation session (e.g., active electrical stimulation session, sham stimulation session, as shown in FIGURE 5A, etc.) and/or any other suitable parameters associated with the stimulation session and waveforms included in the stimulation session. The parameters can include any or all of: a type of waveform, a type of electrical stimulation, an intensity level (e.g., current amplitude) of electrical stimulation applied during the stimulation session, an activity type and/or goal (e.g., athletic activity, music, focus, memory, mood, etc.) associated with the electrical stimulation, a duration of stimulation, and/or any other suitable parameters.

Additionally or alternatively, one or more inputs associated with a user can be received in S210, such as any or all of: user stimulation preferences (e.g., stored at a client application, stored in remote storage, etc.), user information (e.g., demographic information), user historical data (e.g., stimulation session history, task performance data, etc.), and/or any other suitable information. Further additionally or alternatively, any other suitable information (e.g., environmental information from one or more sensors of the system) can be received.

S210 is preferably performed at least once during the method 200 prior to the subsequent processes of the method. S210 can additionally be performed multiple times throughout the method 200, such as any or all of the following: any time that a user requests an electrical stimulation session to be applied, each time that a user requests adjustment (e.g., pausing, stopping, adjustment of intensity, etc.) of the electrical stimulation, and/or at any other suitable time(s) during the method 200.

S210 preferably includes passing an electrical stimulation plan to a subsequent process of the method 200 wherein the electrical stimulation plan is further analyzed and/or adjusted in the subsequent processes of the method 200. Additionally or alternatively, S210 can include retaining a temporary or persistent record of the input (e.g., originally requested action, adjusted electrical stimulation session, etc.). The record can be retained in temporary and/or permanent storage, which can be arranged at any or all of: the electrical stimulation device (e.g., onboard the head apparatus), at a client application and/or user device executing the client application, at remote storage (e.g., associated with a remote computing subsystem), distributed among multiple components, and/or at any other suitable component(s).

In a first variation, an input is received from the user at the client application, wherein the input includes a request from the user to begin an electrical stimulation session. Additionally, one or more inputs can be received subsequently in method, such as while stimulation is being applied. In a specific example, the subsequent inputs can be received either at the head apparatus (e.g., set of buttons onboard the head apparatus) or at the client application.

### 4.2 Method: Requesting a suspension of electrical stimulation in the electrical stimulation plan S220

The method 200 can include requesting a suspension of electrical stimulation in the electrical stimulation plan S220, which can function to implement a user's pausing (e.g., as received as an input in S210) of electrical stimulation, prevent a user from experiencing a transition between waveforms, prevent a user from experiencing a large voltage change, and/or perform any other suitable function.

Requesting a suspension S220 includes passing a request for zero stimulation output (e.g., zero current amplitude) to subsequent processes of the method 200 (e.g., passing a request for zero output down on the stimulation stack of process blocks). S220 can be performed based on any or all of: working data (e.g., set by requests from other blocks or as an effect of previous items in the stream of requests for action) and/or state information (e.g., set by requests from other blocks or as an effect of previous items in the stream of requests for action), user input (e.g., pressing a button to suspend stimulation, pausing stimulation via a client application, etc.), and/or any other suitable information.

S220 can optionally include determining a type of suspension to be applied to the electrical stimulation plan. The type of suspension can inform, for instance, which of the subsequent blocks may or may not be implemented. Additionally or alternatively, S220 can include recording and/or storing an identifier (e.g., user-requested suspension, system-requested suspension, etc.) associated with the suspension, which gets passed down to one or more subsequent processes of the method. A type of suspension can optionally inform whether or not to monitor a parameter associated with contact between the user and an electrode of the electrical stimulation device, as improper contact (e.g., insufficient contact) can result in any or all of: ineffective stimulation, unsafe stimulation (e.g., full stimulation applied through a small surface area of an electrode, full stimulation applied through only a partial subset of a set of electrodes, etc.), a risk of a user discovering that he is experiencing sham stimulation (e.g., by losing contact with the set of electrodes but still information at the client application that he is receiving electrical stimulation), and/or any other number of effects. In preferred variations, for instance, if a user initiates suspension of electrical stimulation (e.g., through S210), a contact parameter (e.g., impedance) is not monitored during the suspension, whereas if a suspension is triggered as a result of lost contact with the user, a contact parameter is monitored during the suspension (e.g., to determine when proper contact is re-established to begin delivering stimulation).

S220 can be performed after the previous process S210 (e.g., in the event that a request for suspension is passed downward, in the event that a request for suspension is not passed downward, etc.), in absence of the previous process (e.g., in the event that a request for suspension has not been passed downward, in the event that a subsequent process is triggered / requested by the previous process, etc.), multiple times throughout the method (e.g., each time a user pauses stimulation, each time the system suspends stimulation, upon determining that the electrical stimulation session has ended, etc.), and/or at any other suitable times.

In a first variation, S220 includes receiving a request for suspension of stimulation from a user at S210 (e.g., requesting a pause at the client application, requesting a pause at the electrical stimulation device, etc.), wherein S220 includes passing the request downward to a subsequent process (e.g., S230, S240, S250, etc.) of the method 200. In a specific example, the request passed downward is configured to suspend monitoring of a contact parameter (e.g., impedance, in S250, etc.), as the user has triggered the stimulation.

In a second variation additional or alternative to the first variation, S220 includes receiving a request for suspension of stimulation, wherein the request is generated at the system (e.g., at the control subsystem 110, at a processor of the control subsystem 110, at a computing subsystem of the control subsystem, etc.) upon detecting that a contact parameter does not satisfy a contact requirement (e.g., measured impedance is above a predetermined impedance threshold), wherein S220 includes passing the request downward to a subsequent process (e.g., S230, S240, S250, etc.). In a specific example, the request passed downward is configured to initiate and/or maintain monitoring of a contact parameter (e.g., impedance, in S250, etc.), in order to enable detection of proper contact being established (e.g., re-established after user adjusts the electrodes).

In a third variation additional or alternative to any of the previous variations, S220 includes receiving a request for electrical stimulation without suspension, wherein S220 includes passing the request downward to a subsequent process (e.g., S230, S240, S250, etc.) of the method.

In a fourth variation additional or alternative to any of the previous variations, the method 200 is performed in absence of S220. In a specific example, a request in S210 does not include a request for S220 (e.g., and includes a request for a subsequent process).

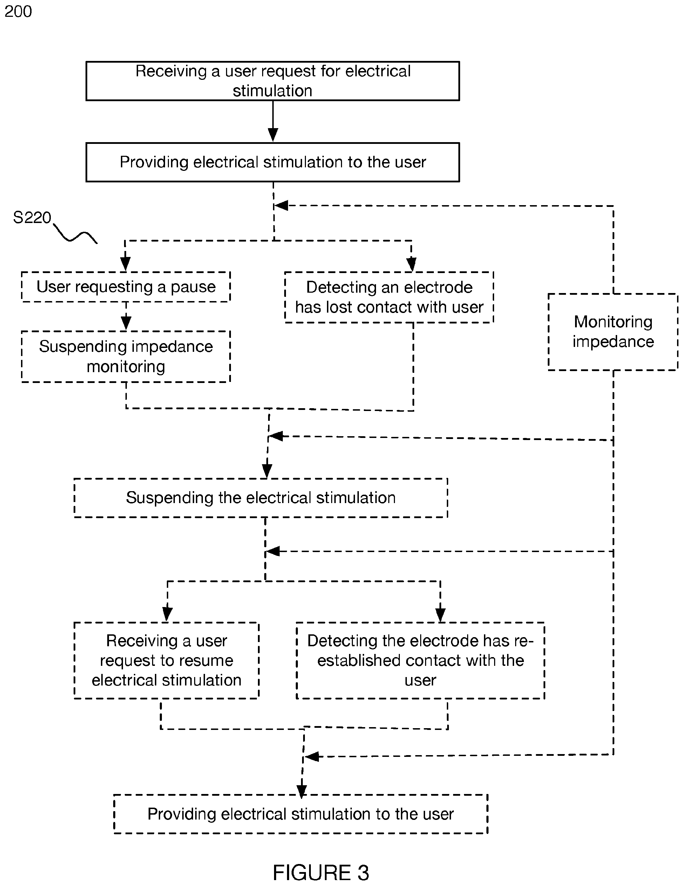

In a fifth variation, S220 is performed as shown in FIGURE 3.

### 4.3 Method: Requesting an adjustment to the electrical stimulation plan S230

The method 200 can include requesting an adjustment to the electrical stimulation plan S230, which functions to adjust one or more stimulation parameters (e.g., current amplitude, duration, voltage, shape of waveform, etc.) of the planned electrical stimulation. Additionally or alternatively, S230 can function to replace a first waveform of the electrical stimulation session with a second waveform, replace a first electrical stimulation session with a second electrical stimulation session, and/or adjust the electrical stimulation plan in any other suitable way.

The stimulation parameters can include any or all of: an amplitude (e.g., current amplitude, voltage amplitude, etc.), frequency, power and/or energy, phase, a waveform shape, a type of electrical stimulation (e.g., tDCS, tRNS, tACS, etc.), a temporal parameter associated with the stimulation (e.g., time at which stimulation begins, time at which stimulation ends, duration, etc.), and/or any other suitable parameters.

S230 can be triggered and/or performed based on any or all of: working data (e.g., set by requests from other blocks or as an effect of previous items in the stream of requests for action) and/or state information (e.g., set by requests from other blocks or as an effect of previous items in the stream of requests for action), user input (e.g., pressing an "increase" button on the electrical stimulation device to increase an intensity and/or amplitude of stimulation, pressing a "decrease" button on the electrical stimulation device to decrease an intensity and/or amplitude of stimulation, selecting or adjusting a stimulation parameter at a client application, etc.), and/or any other suitable information.

An adjustment to one or more stimulation parameters can optionally be received as input in S210, such as in the event that a user selects or adjusts an amplitude of stimulation (e.g., via a client application, via a button on the electrical stimulation device, etc.). Additionally or alternatively, an adjustment to one or more stimulation parameters can be initiated and/or determined by the system 100 (e.g., the control subsystem 110). The system 100 may, for instance, initiate a particular adjustment of stimulation (e.g., slowly ramping up a stimulation amplitude, slowly ramping down a stimulation amplitude, etc.) to comfortably re-initiate stimulation after restoring proper contact, to mitigate an over voltage of scenario, to adjust stimulation while the user interacts with a training task (e.g., synchronizing with a task being performed by the user at a client application).

The system 100 can optionally adjust one or more stimulation parameters (e.g., current amplitude, voltage amplitude, waveform transition, etc.) based on a set of thresholds or other criteria associated with user comfort and/or user safety. An adjustment request received from a user can be implemented with a gradual ramp in order to prevent a transition too abrupt for user comfort from occurring. The thresholds and criteria associated with user comfort can be specific to a user (e.g., gathered from feedback during an onboarding process), general to all users, and/or otherwise determined or defined. Adjusting for user comfort can be additionally or alternatively be performed in accordance with any or all of the embodiments, variations, and examples in U.S. Patent Application Number 15/426,212, filed 07-FEB-2017.

Adjusting the electrical stimulation of the electrical stimulation plan preferably includes requesting the adjustment with one or more of: a multiplicative algorithm, an additive algorithm, a subtractive algorithm, and/or any other suitable algorithm. Adjusting the electrical stimulation of the electrical stimulation plan can additionally or alternatively include replacing a stimulation waveform of a stimulation session, reducing a duration of the electrical stimulation session, increasing a duration of the electrical stimulation session, and/or any other adjustment of the electrical stimulation.

S230 can be performed after the previous process S220 (e.g., in the event that a request for adjustment is passed downward from S210, in the event that a request for adjustment is not passed downward, etc.), in absence of the previous process (e.g., in the event that a request for suspension has not been received, etc.), multiple times throughout the method (e.g., each time a user adjusts stimulation, each time that the system determines an adjustment to stimulation, etc.), and/or at any other suitable times.

S230 can optionally include passing a request for adjustment to the electrical stimulation plan to a subsequent process (e.g., S240, S250, S260, etc.) of the method. Additionally, S230 can include passing and/or storing associated information, such as the initial request for adjustment (e.g., from a user at a client application, determined at the system, etc.), an algorithm or set of rules for adjusting the stimulation, the original electrical stimulation, and/or any other suitable information.

In one variation, S230 includes, in response to receiving a request for adjustment based on a user input received at S210 (e.g., via S220, directly from S210, etc.), determining a gradual ramp to implement the adjustment (e.g., to transition from one stimulation intensity level to another stimulation intensity level) based on an algorithm (e.g., additive algorithm, subtractive algorithm, multiplicative algorithm, etc.); and passing the request for the gradual ramp to a subsequent process of the method 200.

### 4.4 Method: Requesting sham stimulation in the electrical stimulation plan S240

The method 200 can include requesting sham stimulation in the electrical stimulation plan S240, which functions to provide sham stimulation to a user (e.g., without the user's knowledge). Additionally, S240 can enable the use of the system 100 and/or method 200 in a clinical trial, research study, and/or any other suitable environment.

Including sham stimulation in the electrical stimulation plan (e.g., for a portion of the electrical stimulation plan, for all of the electrical stimulation plan, etc.) functions to provide an inactive form (e.g., very weak, very brief, zero amplitude, etc.) of electrical stimulation to the user. The sham stimulation can induce a placebo effect in the user, where the user believes that he or she is receiving an active form of electrical stimulation; additionally or alternatively, the sham stimulation can cause any other suitable effect(s).

The sham stimulation preferably includes an initial non-zero amplitude of stimulation (e.g., which corresponds an amplitude initially planned in S210 or an adjusted stimulation in S230), wherein the initial amplitude decreases to zero (e.g., through a ramping down, through a gradual ramping down, etc.) until the sham stimulation is about to end, at which time the amplitude increases (e.g., to the initial maximum value, to another value, etc.) and then decreases to zero to end the sham stimulation. The initial spike and the end spike in stimulation can trick the user into believing that he or she is receiving substantially non-zero stimulation for the duration of the electrical stimulation session. The portion the electrical stimulation session between the initial spike and the end spike is preferably prescribed to have zero current amplitude, but can additionally or alternatively be prescribed to have a minimal current amplitude (e.g., less than 10% of the maximum current amplitude in one or both of the initial spike and the end spike, less than 10% of the current amplitude provided by a non-sham stimulation counterpart, etc.), or be otherwise configured. Further additionally or alternatively, the sham stimulation can be otherwise defined and/or prescribed.

S240 can be triggered and/or performed based on any or all of: working data (e.g., set by requests from other blocks or as an effect of previous items in the stream of requests for action) and/or state information (e.g., set by requests from other blocks or as an effect of previous items in the stream of requests for action), user input (e.g., a researcher and/or private investigator of a clinical trial triggering sham stimulation at a client application, a researcher and/or private investigator of a clinical trial triggering sham stimulation at a remote computing system, a researcher and/or private investigator of a clinical trial triggering sham stimulation at the electrical stimulation device, etc.), and/or any other suitable information. Additionally or alternatively, S240 can be triggered by the system 100 (e.g., at the control subsystem 110, at a computing subsystem of the control subsystem 110, randomly, at electrical stimulation devices of a predetermined set of participants of a clinical trial, etc.).

In some variations, for instance, a request for sham stimulation can be received as an input in S210 (e.g., at a client application of a user device, at a remote computing subsystem, etc.), such as in the event that a researcher or investigator of a clinical trial requests that a participant receive sham stimulation.

S240 can optionally include determining (e.g., calculating, retrieving from a lookup table or database, etc.) one or parameters associated with the sham stimulation, such as any or all of: a current amplitude associated with an initial spike of stimulation, a current amplitude associated with a final spike of stimulation, a current amplitude between the initial spike and the final spike, a parameter (e.g., duration of time, slope, etc.) associated with a ramp down in current amplitude, a parameter (e.g., duration of time, slope, etc.) associated with a ramp up in current amplitude, a waveform shape, and/or any other suitable parameter(s). Additionally or alternatively, the parameters associated with sham stimulation can be predetermined (e.g., fixed, constant, stored in memory, etc.), determined at a later process of the method 200, and/or otherwise determined.

S240 can be performed after the previous process S230 (e.g., in the event that a request for sham stimulation is passed downward, in the event that a request for sham stimulation is not passed downward, etc.), in absence of the previous process (e.g., in the event that a request for adjustment has not been received, in the event that a request for sham stimulation is coded in the initial electrical stimulation plan, etc.), multiple times throughout the method (e.g., each time a user requests sham stimulation be applied, for the duration of sham stimulation, etc.), and/or at any other suitable times.

In variations where S240 is performed after S230, S240 functions to have the effect of shamming stimulation output in a manner that preserves behavior of the adjuster and other process blocks as if the stimulation was being delivered normally. In other words, the placement of S240 after S230 can function to preserve the behavior of an adjuster block, as it can ensure any or all of: an initial spike of the sham stimulation has an amplitude corresponding to an adjusted amplitude (e.g., received by the user, generated at the system, etc.); a final spike of the sham stimulation has an amplitude corresponding to an adjusted amplitude (e.g., received by the user, generated at the system, etc.); a display of information at a user interface (e.g., client application) that corresponds to the adjusted stimulation (e.g., to prevent a user from discovering that sham stimulation is being provided); and/or any other suitable output.

S240 optionally includes passing a request for sham stimulation to a subsequent process (e.g., S250, S260, etc.) of the method. Additionally or alternatively, S240 can include passing downward and/or storing associated information, such as the initial request for sham stimulation (e.g., from a user at a client application, determined at the system, etc.), passing downward and/or storing a set of parameters associated with the sham stimulation, passing downward and/or storing the original electrical stimulation or an intermediate electrical stimulation (e.g., in the event that sham stimulation is not triggered), and/or any other suitable information.

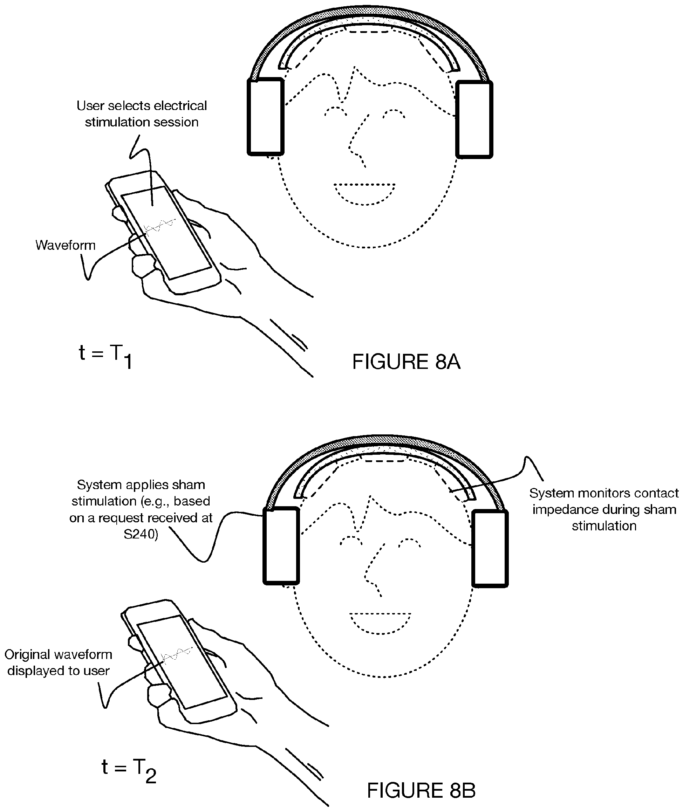

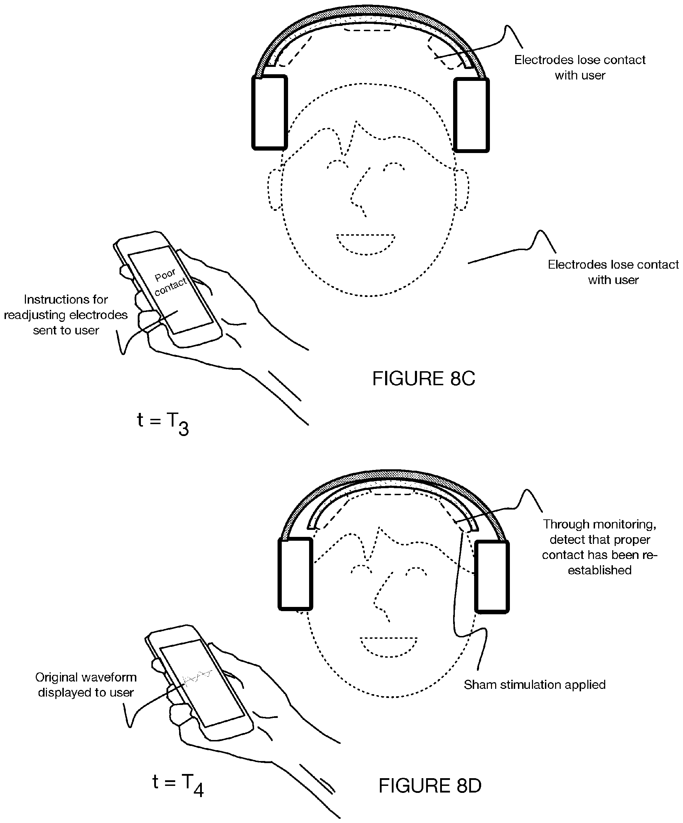

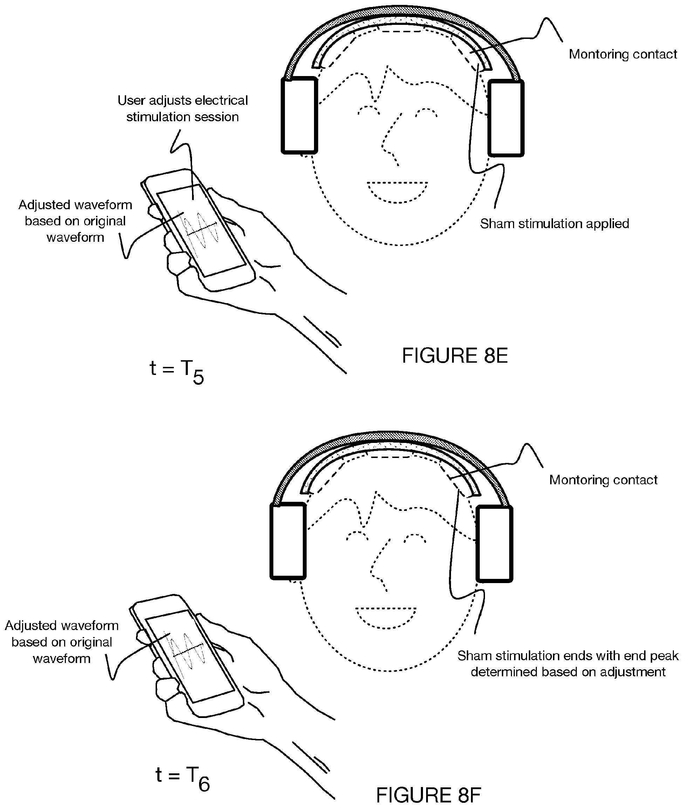

In a first variation of S240 (e.g., as shown in FIGURES 8A-8F), wherein a user makes a selection for non-sham stimulation at S210, a request for sham stimulation is made at S240 (e.g., based on a request generated randomly at the system for a randomized study, based on a request from a private investigator of a research study, etc.), wherein the request for sham stimulation results in a request for monitoring at a subsequent process block.

### 4.5 Requesting monitoring of the electrical stimulation plan S250

The method 200 can include requesting monitoring of the electrical stimulation plan S250, which functions to check if the electrical stimulation plan is able to be monitored for one or more parameters (e.g., impedance) associated with contact between the user and an electrode of the electrical stimulation device. Additionally or alternatively, S250 can function to enable the electrical stimulation plan to be monitored (e.g., through implementation of a set of biphasic current pulses), allowing the electrical stimulation plan to proceed in absence of monitoring, preventing the electrical stimulation plan from including monitoring, and/or performing any other suitable function.

The set of one or more parameters being monitored preferably includes an impedance associated with one or more of the electrodes in the electrical stimulation device, wherein the value of the impedance indicates a level of contact between the one or more electrodes and the user (e.g., skin of the user, scalp of the user, etc.). The impedance can optionally be measured and monitored in accordance with any or all of the variations, embodiments, and examples described in U.S. Patent Application Number 15/426,212, filed 07-FEB-2017. Additionally or alternatively, the impedance can be otherwise measured. Further additionally or alternatively, the set of parameters can include any suitable parameters (e.g., associated with contact with a user, independent of contact with a user, etc.), such as any or all of: an amount of charge delivered through one or more electrodes, a surface area of one or more electrodes through which charge is being delivered, a usage of one or more electrodes, and/or any other suitable parameters.

S250 can be performed after the previous process S240 (e.g., in the event that a request for sham stimulation is passed downward, in the event that a request for sham stimulation is not passed downward, etc.), in absence of the previous process (e.g., in the event that a request for adjustment has not been received, in the event that a request for sham stimulation is coded in the initial electrical stimulation plan, etc.), multiple times throughout the method (e.g., each time a user requests for sham stimulation to be applied, for the duration of sham stimulation, etc.), and/or at any other suitable times.

Triggering a monitoring of one or more parameters can occur in response to a request passed downward from S240 and/or previous processes of the method (e.g., S230, S220, S210, etc.). In some variations, for instance, S250 includes receiving a request for sham stimulation from S240, which triggers integrating a monitoring plan into the electrical stimulation (e.g., by passing a request for monitoring downward in the method 200).

The monitoring plan can include the interleaving of waveform and/or an alternate waveform into the sham stimulation, such as a set of biphasic pulses (e.g., biphasic current pulses), which are applied during (e.g., interleaved into, superimposed, etc.) a zero amplitude portion (e.g., the portion between the initial spike and the final spike) of the sham stimulation. Additionally or alternatively, biphasic pulses can be applied at any suitable portion of an electrical stimulation session (e.g., at a nonzero stimulation portion), the monitoring plan can include any other suitable alteration of a waveform,

In some variations, the monitoring plan includes one or more of the variations, embodiments, and examples for monitoring an electrical stimulation session described in U.S. Patent Application Number 15/426,212, filed 07-FEB-2017. In a specific example, the request includes a monitoring plan that interleaves an alternate waveform such as single or multiple biphasic pulses to allow determination of a calculated parameter (e.g., impedance).

A monitoring plan is preferably integrated into the electrical stimulation plan in variations in which sham stimulation is applied to the user. This can function, for instance, to prevent a user from realizing that sham stimulation is being applied. In examples of these variations, the monitoring plan enables a user experiencing sham stimulation to be notified (e.g., through a notification at the client application, as shown in FIGURE 5B, through a notification at the electrical stimulation device, etc.) in the event that proper contact is lost (e.g., an electrode slips from the user's scalp, the user takes the electrode stimulation device off of his or her head, etc.), even when substantially zero stimulation (e.g., zero current amplitude, etc.) is being applied.

A monitoring plan is preferably not integrated into the electrical stimulation session in variations in which a request for suspension of stimulation has been initiated in S220 and passed downward to S250 (e.g., directly, via intermediate processes, etc.), as monitoring is likely not required while electrical stimulation has been suspended (e.g., intentionally suspended). In variations where a request for stimulation has been initiated, S250 can optionally be skipped (e.g., absent from the method 200). Alternatively, S250 can be performed and include any or all of: no action or adjustment to the request for electrical stimulation; the addition of an identifier corresponding to "no monitoring" added to request for electrical stimulation; and/or any other suitable output.

A monitoring plan can optionally be integrated into the electrical stimulation session in variations in which no request for suspension of stimulation has been initiated and in which no request for sham stimulation has been initiated. In these variations, a monitoring plan based on the electrical stimulation plan alone (e.g., without an interleaved waveform) can be implemented. In preferred variations, for instance, a contact parameter (e.g., impedance) can be monitored based on the current waveform without requiring an interleaved waveform (e.g., set of biphasic current pulses). Additionally or alternatively, a monitoring plan as described above can be implemented, a monitoring plan that accounts for times at which the waveform momentarily has zero amplitude (e.g., waveform crossing an x-axis) can be implemented, no monitoring plan can be implemented, and/or a request for any other electrical stimulation plan can be produced as an output.

Additionally or alternatively, a monitoring plan can be included in any suitable electrical stimulation plan, be absent from an electrical stimulation session including sham stimulation, and/or be otherwise implemented.

S250 optionally includes passing a request for monitoring to a subsequent process (e.g., S260, etc.) of the method. Additionally or alternatively, S250 can include passing and/or storing any or all associated information, such as the initial request for sham stimulation (e.g., from a user at a client application, determined at the system, etc.), passing downward and/or storing a set of parameters associated with the sham stimulation, passing downward and/or storing the original electrical stimulation or an intermediate electrical stimulation (e.g., in the event that sham stimulation is not triggered), and/or any other suitable information.

In variations where a sham stimulation plan is implemented in S240, performing S250 after S240 (as opposed to before S240) can function to prevent a monitoring plan in S250 from being removed (e.g., erased, deleted, overridden, etc.) when implementing the sham stimulation plan. In examples, for instance, implementing the sham stimulation plan includes overriding at least a middle portion of an electrical stimulation waveform and replacing it with zero stimulation (e.g., zero current amplitude). Implementing this would override the monitoring plan, preventing one or more parameters (e.g., impedance) from being monitored.

In a first variation of S250, wherein no suspension of stimulation has been requested in S220 and wherein a sham stimulation plan has been requested in S240, S250 includes requesting the implementation of a monitoring plan configured to monitor a contact parameter while the sham stimulation is being applied to the user. In a specific example, a request resulting from S250 (e.g., new request, appended request, etc.) includes a plan for interleaving a set of biphasic current pulses into a zero amplitude portion of the sham stimulation.

In a second variation of S250, wherein a suspension of stimulation has been requested in S220 (e.g., and wherein a sham stimulation plan has been requested in S240, and wherein no sham stimulation plan has been requested in S240, etc.), S250 includes making no request for monitoring.

In a third variation of S250, wherein no sham stimulation is requested and no request for suspension of stimulation has been implemented, a monitoring plan based on the current waveform in the electrical stimulation plan is requested. In a specific example, an impedance value is monitored based on the current waveform being applied as part of an active electrical stimulation plan.

### 4.6 Method: Requesting a trim adjustment to the electrical stimulation plan S260

The method 200 can optionally include requesting a trim adjustment to the electrical stimulation plan S260, which functions to prepare the electrical stimulation plan for use with a particular electrical stimulation device. Additionally, S250 can function to overcome one or more limitations associated with hardware used in the electrical stimulation device, such as an imperfect DAC component.

S260 can be triggered and/or performed based on any or all of: working data (e.g., set by requests from other blocks or as an effect of previous items in the stream of requests for action) and/or state information (e.g., set by requests from other blocks or as an effect of previous items in the stream of requests for action), calibration data (e.g., non-volatile calibration data,, volatile calibration data, etc.), user and/or manufacturer input (e.g., entry of calibration data, entry of DAC performance data, etc.), and/or any other suitable information. Additionally or alternatively, S260 can be triggered by the system 100 (e.g., at the control subsystem 110, at a computing subsystem of the control subsystem 110, randomly, at electrical stimulation devices of a predetermined set of participants of a clinical trial, etc.). Further additionally or alternatively, the method 200 can be performed in absence of S260.

S260 can include requesting an adjustment, such as a multiplicative and/or additive and/or subtractive adjustment of one or more stimulation parameters (e.g., current amplitude, voltage, etc.) of the electrical stimulation plan, wherein the adjustment is configured to compensate for a variability in output stage hardware performance of one or more components (e.g., DAC converter) of the system 100.

In one variation, S260 includes applying a linear trim algorithm to a waveform of the electrical stimulation plan to account for a sub-optimal or otherwise configured DAC component. In a specific example, for a system having a DAC component known from non-volatile calibration data to result in a predetermined additional current amplitude (e.g., 0.05 mA) to the waveform, S260 includes subtracting out the predetermined value from the electrical stimulation plan.

### 4.7 Method: Delivering electrical stimulation based on the electrical stimulation plan S270

The method 200 can include delivering electrical stimulation based on the electrical stimulation plan S270, which functions to apply electrical stimulation to the user in accordance with the electrical stimulation plan determined in any or all of S210-S260.

S270 preferably includes transmitting the electrical stimulation plan to a DAC component of the system, such as through any or all of the variations, embodiments, and examples described in P08, which is incorporated in its entirety herein by this reference.

In one variation, S270 includes applying electrical stimulation through a set of electrodes of an electrical stimulation device, wherein applying the electrical stimulation includes transmitting a digital electrical stimulation plan produced through any or all of the processes described above to a DAC of the electrical stimulation device, wherein the DAC transforms a digital waveform definition of the electrical stimulation plan to an analog output (e.g., to an output stage that comprises a voltage-controlled current source including an amplifier operable to take an input voltage from the DAC and produce a controlled current through the stimulation plan in proportion to the input voltage, to the electrodes of the electrical stimulation device used for stimulation).

### 4.8 Method: Verifying the electrical stimulation plan S280

The method 200 can optionally including verifying the electrical stimulation plan S280, which functions to check that the electrical stimulation provided by the electrical stimulation device substantially agrees with (e.g., is equal to, is within a predetermined set of thresholds, etc.) the electrical stimulation specified by the electrical stimulation plan. Additionally or alternatively, S280 can function to check a request determined at any or all of the process block(s) described above with a desired request, a previous request (e.g., a request associated with previously applied electrical stimulation), and/or any other suitable requests or actual applied electrical stimulation parameters.

S280 is preferably performed after (e.g., in response to) S270, but can additionally or alternatively be performed prior to S270, at multiple times throughout the method 200, and/or the method can be performed in absence of S280.

In one variation of S280, S280 includes regularly comparing a parameter (e.g., current amplitude value, maximum current amplitude value, etc.) measured based on the output electrical stimulation applied at the electrical stimulation device with the prescribed value of the parameter requested a process block (e.g., monitoring process block).

### 4.9 Method: Providing an output to a user S290

The method 200 can include providing an output to a user S290, which functions to inform a user of any or all of: an electrical stimulation session being applied to the user, an electrical stimulation session to be perceived by the user (e.g., in the event that the user is receiving sham stimulation), an instruction for the user to receive proper electrical stimulation, and/or any other suitable information.

The user preferably includes the wearer of the electrical stimulation device but can additionally or alternatively include a user associated with providing the electrical stimulation (e.g., a researcher, a private investigator of a clinical trial, a clinician, etc.), a user who selects the electrical stimulation session, and/or any other suitable users.

The output can be provided at any or all of: a user device (e.g., at a client application executing on the user device), the electrical stimulation device (e.g., through a haptic output, through an audio output, through a visual output, etc.), and/or at any other suitable user interface.

The output preferably includes information associated with an electrical stimulation session (e.g., one or more waveforms included in the electrical stimulation session, one or more stimulation parameters associated with the electrical stimulation session, one or more temporal parameters associated with the electrical stimulation session, etc.), wherein the electrical stimulation session can include any or all of: an electrical stimulation session actively being applied to the user, an electrical stimulation session configured to be and/or desired (e.g., by a researcher) to be perceived by the user (e.g., in the event that the user is experiencing sham stimulation), a sham stimulation session, an electrical stimulation session and/or sham stimulation session previously applied to a user, an electrical stimulation session and/or sham stimulation session planned to be applied to a user (e.g., at a future time point), and/or any other suitable information. Additionally or alternatively, the output can include any or all of: contact information (e.g., indicating that an electrode has lost contact with the user, indicating that an electrode is oriented improperly, etc.), instructions (e.g., guiding a user to proper placement of the electrical stimulation device, etc.), task information (e.g., of a task performed during an electrical stimulation session, of a task performed prior to an electrical stimulation session, of a task performed after an electrical stimulation session, etc.), user information, a power and/or charge status of the electrical stimulation device, and/or any other suitable information.

In variations where the user is receiving sham stimulation (e.g., without the user's knowledge), the output preferably reflects an electrical stimulation session (e.g., includes electrical stimulation session parameters) configured to perceived by the user (e.g., originally selected by the user, originally communicated to the user, etc.), but can additionally or alternatively reflect the sham stimulation session and/or include any other suitable output. In a specific example, for instance, an indication of an active electrical stimulation session and/or one or parameters (e.g., current amplitude) associated with the active electrical stimulation session is displayed at a client application executing on the user device.

The output can be provided through any or all of: a message (e.g., text message, message at the client application, etc.), a display of the user interface (e.g., via the client application), a call (e.g., at the user device), a visual output (e.g., at the client application, at the user device, at a display of the electrical stimulation device, at an indicator light of the electrical stimulation device, etc.), an audio output (e.g., at the user device, at the electrical stimulation device, etc.), a haptic output (e.g., at the user device, at the electrical stimulation device, etc.), and/or any other suitable output can be provided.

In a first variation, the output includes a stimulation parameter associated with the electrical stimulation session, wherein the parameter is provided at a client application executing on the user device. In a specific example, the parameter includes a value (e.g., 2mA) and/or a level (e.g., low, medium, high, etc.) of a current amplitude of the electrical stimulation.

In a second variation, the output includes a depiction (e.g., schematic) of a waveform of the electrical stimulation session being applied to the user. In a specific example, the waveform is shown at a display of the user device via the client application. 4.10 Method: Repeating the above S295

The method 200 can include repeating any or all of the above processes, which can function to continuously analyze and process the electrical stimulation (e.g., active electrical stimulation, sham stimulation, etc.) being applied to the user.

S295 preferably includes repeating any or all of S210-S290 (e.g., S210-S270, a subset of S210-S270, etc.) throughout out the duration of an electrical stimulation session, such as any or all of: continuously, intermittently, at a predetermined frequency (e.g., once every 0.4 milliseconds, more than once every 0.4 milliseconds, once every millisecond, more than once every millisecond, once per second, any frequency between 10 times per second and once every five minutes, more than 10 times per second, less than once every five minutes, etc.), at random intervals, in response to one or more triggers (e.g., received by the user, generated at the system, detected by a sensor subsystem of the system, etc.), and/or at any other suitable time(s).

In some variations, S295 includes repeating a first subset of one or more processes at a predetermined frequency and repeating a second subset of one or more processes in response to a trigger. In a specific example, for instance, S295 includes repeating any or all of S210-S280 at a predetermined frequency and performing S290 in response to a trigger (e.g., lost contact between the electrical stimulation device and the user).

### 4.11 Method: Variations

In one variation of the method 200, the method includes: receiving a request for electrical stimulation (e.g., from a client application, from a button of the electrical stimulation device, from a remote computing system, from an onboard computing system, etc.) at the control system (e.g., onboard the electrical stimulation device), wherein the request for electrical stimulation is associated with and/or triggers an electrical stimulation plan; at the processing system, checking for a set of requests, wherein each of the set of requests is checked for at a process block of the stimulation stack; modifying the electrical stimulation plan based on which, if any, of the process blocks receives a request; providing electrical stimulation (e.g., active electrical stimulation, sham stimulation, etc.) to the user based on the modified electrical stimulation plan (or the original electrical stimulation plan if no requests are received); and repeating any or all of the above. Additionally, the method can include providing and/or modifying (e.g., based on the modified electrical stimulation plan, based on the original electrical stimulation plan in the case of sham stimulation, etc.) a graphic or other information (e.g., current amplitude value, stimulation intensity, stimulation duration, etc.) to a user at a display of the user device. Additionally or alternatively, the method can include any other suitable processes performed in any suitable order.

Although omitted for conciseness, the preferred embodiments include every combination and permutation of the various system components and the various method processes, wherein the method processes can be performed in any suitable order, sequentially or concurrently.

As a person skilled in the art will recognize from the previous detailed description and from the figures and claims, modifications and changes can be made to the preferred embodiments of the invention without departing from the scope of this invention defined in the claims and following statements.

In one aspect of interest there is provided a method for delivering electrical stimulation to a user through an electrical stimulation device, the method comprising: at the electrical stimulation device, receiving a request for electrical stimulation, wherein the request is associated with an electrical stimulation plan, wherein the electrical stimulation plan comprises a set of stimulation parameters; at processing system associated with the electrical stimulation device: receiving the electrical stimulation plan; checking for each of a set of requests, wherein the set of requests comprises: a first request, wherein the first request comprises a request for a suspension of stimulation; a second request, wherein the second request comprises an adjustment of at least one of the set of stimulation parameters; and a third request, wherein the third request comprises a request for sham stimulation; determining a modified electrical stimulation plan based on checking for each of the set of requests; at the electrical stimulation device, providing electrical stimulation based on the modified electrical stimulation plan to the user. In one form the processing system may be onboard the electrical stimulation device.

In another form, in an event that the second request is received, the method may further comprise monitoring a contact parameter associated with contact between the electrical stimulation device and the user. In this case, in an event that the contact parameter is below a predetermined threshold, a notification may be provided to the user at a client application executing on a user device, wherein the user device is in communication with the electrical stimulation device.

The method above, wherein the first request is checked prior to the second request, and wherein the second request is checked prior to the third request. The method above also may further comprise checking for a fourth request, wherein the fourth request comprises a second adjustment of the set of stimulation parameters. In this case, the second adjustment may be determined based on a feature of a component of the electrical stimulation device. The predetermined frequency may be at least once every millisecond.

## Claims

1. A system for delivering electrical stimulation to a user through an electrical stimulation device, the system comprising:
a control system (110), wherein the control system (110) comprises a processing system that includes a stimulation stack (120) and the control system is configured to implement the stimulation stack (120);
an electrical stimulation device (130);
a user device (140), wherein the user device (140) is in communication with the electrical stimulation device;
a client application (150), wherein the client application is configured to be executed by the user device (140);
wherein the client application (150) is configured to:
o receive an input from a user, wherein the input comprises a selection of an electrical stimulation plan, wherein the electrical stimulation plan comprises a set of stimulation parameters; and
o transmit the electrical stimulation plan to a processing system associated with the electrical stimulation device;
and wherein the stimulation stack (120) is configured during a single stimulation session to:
o at a predetermined frequency during the stimulation session, check for each of a set of requests based on the electrical stimulation plan, wherein the set of requests comprises:
▪ a first request, wherein the first request comprises a request for a suspension of stimulation;
▪ a second request, wherein the second request comprises an adjustment of at least one of the set of stimulation parameters; and
▪ a third request, wherein the third request comprises a request for sham stimulation; and
o determine a modified electrical stimulation plan based on the check for each of the set of requests;
and wherein the electrical stimulation device (130) is configured to provide electrical stimulation based on the modified electrical stimulation plan to the user.

2. The system of claim 1, wherein the processing system is arranged to be onboard the electrical stimulation device.

3. The system of claim 1, wherein the first request is received from the client application.

4. The system of claim 1, wherein the set of stimulation parameters includes a current amplitude.

5. The system of claim 1, wherein in an event that the second request is received, the stimulation stack (120) is further configured to monitor a contact parameter associated with contact between the electrical stimulation and the user.

6. The system of claim 5, wherein the stimulation stack (120) is further configured to, in an event that the contact parameter is below a predetermined threshold, provide a notification to the user at the client application.

7. The system of claim 1, wherein the first request is checked prior to the second request, and wherein the second request is checked prior to the third request.

8. The system of claim 1, wherein the stimulation stack (120) is further configured to check for a fourth request, wherein the fourth request comprises a second adjustment of the set of stimulation parameters.

9. The system of claim 8, wherein the stimulation stack (120) is configured to determine the second adjustment based on a feature of a component of the electrical stimulation device.

10. The system of claim 9, wherein the component comprises a digital-to-analog converter.

11. The system of claim 10, wherein the predetermined frequency is at least once every millisecond.

12. The system of claim 1, further comprising the client application being configured to provide a visual graphic, wherein the visual graphic indicates at least one of a set of modified parameters of the modified electrical stimulation plan.

## Patentansprüche

1. Ein System zum Liefern von elektrischer Stimulation an einen Benutzer durch eine Vorrichtung zur elektrischen Stimulation, wobei das System Folgendes beinhaltet:
ein Steuersystem (110), wobei das Steuersystem (110) ein Verarbeitungssystem beinhaltet, das einen Stapel (120) zur Stimulation umfasst, und das Steuersystem konfiguriert ist, um den Stapel (120) zur Stimulation zu implementieren;
eine Vorrichtung (130) zur elektrischen Stimulation;
eine Benutzervorrichtung (140), wobei die Benutzervorrichtung (140) in Kommunikation mit der Vorrichtung zur elektrischen Stimulation steht;
eine Client-Anwendung (150), wobei die Client-Anwendung konfiguriert ist, um durch die Benutzervorrichtung (140) ausgeführt zu werden;
wobei die Client-Anwendung (150) für Folgendes konfiguriert ist:
o Empfangen einer Eingabe von einem Benutzer, wobei die Eingabe eine Auswahl eines Plans zur elektrischen Stimulation beinhaltet, wobei der Plan zur elektrischen Stimulation einen Satz von Stimulationsparametern beinhaltet; und
o Übertragen des Plans zur elektrischen Stimulation an ein Verarbeitungssystem, das mit der Vorrichtung zur elektrischen Stimulation assoziiert ist;
und wobei der Stapel (120) zur Stimulation während einer einzelnen Simulationssitzung für Folgendes konfiguriert ist:
o bei einer vorbestimmten Frequenz während der Stimulationssitzung, Überprüfen auf jeden eines Satzes von Anfragen basierend auf dem Plan zur elektrischen Stimulation, wobei der Satz von Anfragen Folgendes beinhaltet:
▪ eine erste Anfrage, wobei die erste Anfrage eine Anfrage nach einer Suspension der Stimulation beinhaltet;
▪ eine zweite Anfrage, wobei die zweite Anfrage eine Anpassung von mindestens einem des Satzes von Stimulationsparametern beinhaltet; und
▪ eine dritte Anfrage, wobei die dritte Anfrage eine Anfrage nach einer Stimulation einer Täuschung beinhaltet; und
o Bestimmen eines modifizierten Plans zur elektrischen Stimulation basierend auf dem Überprüfen auf jede des Satzes von Anfragen;
und wobei die Vorrichtung (130) zur elektrischen Stimulation konfiguriert ist, um elektrische Stimulation basierend auf dem modifizierten Plan zur elektrischen Stimulation an den Nutzer bereitzustellen.

2. System gemäß Anspruch 1, wobei das Verarbeitungssystem eingerichtet ist, um sich in der Vorrichtung zur elektrischen Stimulation eingebaut zu befinden.

3. System gemäß Anspruch 1, wobei die erste Anfrage von der Client-Anwendung empfangen wird.

4. System gemäß Anspruch 1, wobei der Satz von Stimulationsparametern eine Stromamplitude umfasst.

5. System gemäß Anspruch 1, wobei bei einem Ereignis, dass die zweite Anfrage empfangen wird, der Stapel (120) zur Stimulation ferner konfiguriert ist, um einen Kontaktparameter zu überwachen, der mit Kontakt zwischen der elektrischen Stimulation und dem Benutzer assoziiert ist.

6. System gemäß Anspruch 5, wobei der Stapel (120) zur Stimulation ferner konfiguriert ist, um bei einem Ereignis, dass sich der Kontaktparameter unter einem vorbestimmten Schwellenwert befindet, eine Benachrichtigung an den Benutzer an der Client-Anwendung bereitzustellen.

7. System gemäß Anspruch 1, wobei die erste Anfrage vor der zweiten Anfrage überprüft wird, und wobei die zweite Anfage vor der dritten Anfrage überprüft wird.

8. System gemäß Anspruch 1, wobei der Stapel (120) zur Stimulation ferner konfiguriert ist, um auf eine vierte Anfrage zu überprüfen, wobei die vierte Anfrage eine zweite Anpassung des Satzes von Stimulationsparametern beinhaltet.

9. System gemäß Anspruch 8, wobei der Stapel (120) zur Stimulation konfiguriert ist, um die zweite Anpassung basierend auf einem Merkmal einer Komponente der Vorrichtung zur elektrischen Stimulation zu bestimmen.

10. System gemäß Anspruch 9, wobei die Komponente einen Digital-Analog-Wandler beinhaltet.

11. System gemäß Anspruch 10, wobei die vorbestimmte Frequenz mindestens einmal jede Millisekunde ist.

12. System gemäß Anspruch 1, das ferner die Client-Anwendung beinhaltet, die konfiguriert ist, um eine visuelle Graphik bereitzustellen, wobei die visuelle Graphik mindestens einen eines Satzes von modifizierten Parametern des modifizierten Plans zur elektrischen Stimulation anzeigt.

## Revendications

1. Un système pour administrer une stimulation électrique à un utilisateur par le biais d'un dispositif de stimulation électrique, le système comprenant :
un système de commande (110), le système de commande (110) comprenant un système de traitement qui inclut une pile de stimulation (120) et le système de commande étant configuré pour implémenter la pile de stimulation (120) ;
un dispositif de stimulation électrique (130) ;
un dispositif utilisateur (140), le dispositif utilisateur (140) étant en communication avec le dispositif de stimulation électrique ;
une application client (150), l'application client étant configurée pour être exécutée par le dispositif utilisateur (140) ;
dans lequel l'application client (150) est configurée pour :
o recevoir une entrée en provenance d'un utilisateur, l'entrée comprenant une sélection d'un plan de stimulation électrique, le plan de stimulation électrique comprenant un ensemble de paramètres de stimulation ; et
o transmettre le plan de stimulation électrique à un système de traitement associé au dispositif de stimulation électrique ;
et dans lequel la pile de stimulation (120) est configurée pendant une séance de stimulation unique pour :
o à une fréquence prédéterminée pendant la séance de stimulation, effectuer une vérification pour chaque requête d'un ensemble de requêtes sur la base du plan de stimulation électrique, l'ensemble de requêtes comprenant :
▪ une première requête, la première requête comprenant une requête de suspension de stimulation ;
▪ une deuxième requête, la deuxième requête comprenant un réglage d'au moins un paramètre de l'ensemble de paramètres de stimulation ; et
▪ une troisième requête, la troisième requête comprenant une requête de stimulation fictive ; et
o déterminer un plan de stimulation électrique modifié sur la base de la vérification effectuée pour chaque requête de l'ensemble de requêtes ;
et dans lequel le dispositif de stimulation électrique (130) est configuré pour fournir une stimulation électrique sur la base du plan de stimulation électrique modifié à l'utilisateur.

2. Le système de la revendication 1, dans lequel le système de traitement est conçu pour être embarqué sur le dispositif de stimulation électrique.

3. Le système de la revendication 1, dans lequel la première requête est reçue en provenance de l'application client.

4. Le système de la revendication 1, dans lequel l'ensemble de paramètres de stimulation inclut une amplitude de courant.

5. Le système de la revendication 1, dans lequel au cas où la deuxième requête serait reçue, la pile de stimulation (120) est configurée en outre pour surveiller un paramètre de contact associé à un contact entre la stimulation électrique et l'utilisateur.

6. Le système de la revendication 5, dans lequel la pile de stimulation (120) est configurée en outre pour, au cas où le paramètre de contact serait au-dessous d'un seuil prédéterminé, fournir une notification à l'utilisateur au niveau de l'application client.

7. Le système de la revendication 1, dans lequel la première requête est vérifiée avant la deuxième requête, et dans lequel la deuxième requête est vérifiée avant la troisième requête.

8. Le système de la revendication 1, dans lequel la pile de stimulation (120) est configurée en outre pour effectuer une vérification pour une quatrième requête, la quatrième requête comprenant un deuxième réglage de l'ensemble de paramètres de stimulation.

9. Le système de la revendication 8, dans lequel la pile de stimulation (120) est configurée pour déterminer le deuxième réglage sur la base d'une caractéristique d'un composant du dispositif de stimulation électrique.

10. Le système de la revendication 9, dans lequel le composant comprend un convertisseur numérique-analogique.

11. Le système de la revendication 10, dans lequel la fréquence prédéterminée est d'au moins une fois par milliseconde.

12. Le système de la revendication 1, comprenant en outre le fait que l'application client est configurée pour fournir une illustration visuelle, l'illustration visuelle indiquant au moins un paramètre d'un ensemble de paramètres modifiés du plan de stimulation électrique modifié.
